# FASCICULE DE BREVET EUROPEEN

(11) **EP 3 406 288 B1**
(45) Date de publication et mention de la délivrance du brevet: **19.02.2020**
(21) Numéro de dépôt: 18169444.9
(22) Date de dépôt: 26.04.2018
(51) Int. Cl.: A61M 16/16, A61M 16/10

(54) **ENSEMBLE HUMIDIFICATEUR DE GAZ ET VENTILATEUR MÉDICAL À RÉSERVOIR D'EAU À ÉTANCHÉITÉ AMÉLIORÉE**
EINHEIT AUS GASBEFEUCHTER UND MEDIZINISCHEM BEATMUNGSGERÄT MIT WASSERBEHÄLTER MIT VERBESSERTER ABDICHTUNG
GAS HUMIDIFIER ASSEMBLY AND MEDICAL VENTILATOR WITH WATER TANK HAVING IMPROVED SEALING

(30) Priorité: 24.05.2017 FR 1754588
(43) Date de publication de la demande: 28.11.2018
(73) Titulaire: Air Liquide Medical Systems, 92160 Antony (FR)
(72) Inventeur: LEBATTEUR, Nicolas, 91220 BRETIGNY SUR ORGE (FR); MOVSCHIN, Antoine, 75014 PARIS (FR); GUIDUCCI, Hadrien, 75020 PARIS (FR)
(74) Mandataire: Pittis, Olivier

(56) Documents cités:
- EP-A1- 2 848 277
- EP-A2- 2 703 034
- WO-A1-2016/097928

## Description

L'invention concerne un ensemble humidificateur de gaz chauffant /ventilateur médical comprenant un réservoir à eau à couvercle amélioré servant à l'humidification des gaz issus du ventilateur médical.

Habituellement, un dispositif d'humidification de gaz chauffant, généralement appelé humidificateur, comprend un réservoir amovible destiné à contenir un volume d'eau. Ce réservoir est configuré pour permettre le passage d'un débit d'air entre un orifice d'entrée et un orifice de sortie. Son fond comporte une plaque métallique destiné à transmettre à l'eau, de la chaleur provenant d'un boitier chauffant, dans lequel vient se loger le réservoir. Le fond du boitier comporte un élément chauffant relié à ou en contact avec une autre plaque métallique dont le rôle est de transmettre la chaleur produite par l'élément chauffant du boitier à la plaque métallique du réservoir, par l'intermédiaire de la seconde plaque métallique.

Pendant le fonctionnement de l'humidificateur, l'air provenant d'un appareil médical d'assistance respiratoire, i.e. ventilatoire, couramment appelé « ventilateur» médical, entre dans le réservoir, s'y réchauffe et s'y charge en humidité grâce à l'eau qui est chauffée dans le réservoir. L'air chaud humidifié est ensuite acheminé jusqu'aux voies respiratoires d'un patient, via notamment un conduit de gaz flexible alimentant une interface respiratoire, tel un masque respiratoire ou analogue. Un humidificateur de ce type et son fonctionnement sont décrits par EP-A-2848277.

Toutefois, lors de l'utilisation par un patient à domicile, d'un humidificateur chauffant à réservoir à eau, alimenté en gaz, en particulier en air, par un ventilateur pulmonaire à micro soufflante motorisée, il est indispensable de bien maîtriser l'étanchéité du chemin d'air au niveau du réservoir, sans laquelle le traitement thérapeutique délivré par le ventilateur au patient peut ne pas être efficace du fait des fuites de gaz pouvant avoir lieu.

Or, en pratique, il a été constaté des défauts d'étanchéité des joints ou analogues situés au niveau des orifices d'entrée et de sortie d'air, c'est-à-dire aux liaisons entre réservoir à eau et boitier de l'humidificateur, en particulier suite à une insertion par un patient du réservoir à eau dans le boitier de l'humidificateur.

Le problème qui se pose est dès lors de s'assurer que, lorsque le patient insère le réservoir dans le boitier, l'étanchéité soit garantie, notamment au niveau des orifices d'entrée et de sortie de gaz du réservoir, c'est-à-dire de réduire ou d'éliminer les défauts d'étanchéité susceptibles de se produire au niveau desdits orifices d'entrée et de sortie de gaz du réservoir qui se trouvent généralement sur le couvercle.

La solution de l'invention concerne alors un ensemble humidificateur de gaz/ventilateur médical comprenant un ventilateur équipé d'une micro-soufflante électrique et un humidificateur de gaz comprenant un réservoir amovible et un capot, le réservoir comprenant une cuve à eau et un couvercle agencé sur la cuve de manière à former un toit couvrant ladite cuve, caractérisé en ce que :
- le couvercle du réservoir comprend un orifice d'entrée de gaz, un orifice de sortie de gaz, une portion de toit plane contenue dans un plan (P), une première portion de toit inclinée portant l'orifice d'entrée de gaz et formant un premier angle (α) avec le plan (P), où le premier angle (α) est compris entre 15° et 30°, et une seconde portion de toit inclinée portant l'orifice de sortie de gaz et formant un second angle (β) avec le plan (P), où le second angle (β) est compris entre 30° et 50°,
- la cuve à eau comprend un fond muni d'une plaque métallique (appelée « seconde plaque métallique ») et une paroi périphérique se projetant vers le haut à partir dudit fond, ledit couvercle étant solidaire de la paroi périphérique de ladite cuve,
- et le capot comprend un conduit d'évacuation de gaz humidifié en communication fluidique avec l'orifice de sortie de gaz du couvercle du réservoir.

La solution de la présente invention permet de garantir une étanchéité efficace à la fois en entrée et en sortie de réservoir grâce à l'aménagement de portions de toit inclinées sur le couvercle. En effet, grâce aux deux plans inclinés, c'est-à-dire les deux portions de toit inclinées formant entre elle un toit en « V » inversé au sommet du couvercle, lorsque l'utilisateur referme le capot formant la partie haute du boitier sur le réservoir, ce capot exerce sur le réservoir une pression à la fois verticale, pour plaquer le réservoir sur la plaque chauffante située dans le fond du boitier et ainsi optimiser la transmission de chaleur, et horizontale, pour contraindre le ou les joints d'entrée et de sortie d'air du réservoir et donc améliorer l'étanchéité de l'ensemble, et aussi le positionnement du réservoir amovible dans le logement du boitier qui le reçoit.

La présence de plans inclinés est donc particulièrement avantageuse car, contrairement à un couvercle plat, cette architecture particulière permet de mieux maintenir et centrer le réservoir dans le boitier de l'humidificateur et, par ailleurs, d'ajouter un effort sur le réservoir, donc d'améliorer le contact entre les plaques métalliques agencées dans le fond du réservoir et dans le fond du boitier, lesquelles sont chauffées par l'élément chauffant du boitier de l'humidificateur.

Selon le cas, ensemble humidificateur de gaz/ventilateur médical de l'invention peut comprendre l'une ou plusieurs des caractéristiques techniques suivantes :
- la première portion de toit inclinée est plane.
- la seconde portion de toit inclinée est plane.
- la première portion de toit inclinée forme un premier plan P1 incliné selon le premier angle (α) avec le plan P.
- la seconde portion de toit inclinée forme un second plan P2 incliné selon le second angle (β) avec le plan P.
- le plan P est sensiblement parallèle au fond de la cuve.
- la première portion de toit inclinée du réservoir forme, avec le plan P, un premier angle (α) compris entre 15° et 25 °, typiquement de l'ordre de 20°.
- la seconde portion de toit inclinée du réservoir forme, avec le plan P, un second angle (β) compris entre 30 ° et 45 °, typiquement de l'ordre de 40°.
- le couvercle formant un toit fermant la cuve comprend une première portion de toit inclinée et une seconde portion de toit inclinée, lesdites première et seconde portions de toit inclinées formant entre elles un troisième angle (µ) compris entre 90 ° et 150°, de préférence entre 100 ° et 140 °, de préférence encore entre 110 ° et 130 °, typiquement de l'ordre de 120°.
- le couvercle comprend une première portion de toit inclinée et une seconde portion de toit inclinée formant entre elles une partie de toit en « V » inversé.
- l'orifice d'entrée de gaz est en communication fluidique avec un élément de conduit se projetant dans le volume interne de la cuve et comprenant un passage interne de gaz s'étendant entre l'orifice d'entrée de gaz et une bouche de sortie débouchant dans le volume interne de la cuve.
- la cuve à eau est parallélépipédique.
- la cuve à eau a une forme générale de parallélépipède rectangle.
- le couvercle comprend des moyens ou un système de fixation permettant de le solidariser à la paroi périphérique de la cuve, par exemple un système à emboitement.
- le couvercle et/ou la cuve à eau est en polymère, par exemple en plastique du type polypropylène (PP).
- la cuve à eau comprend un fond muni d'une plaque métallique en aluminium, alliage d'aluminium ou acier inoxydable.
- l'élément de conduit comprend un ou plusieurs coudes, de préférence deux coudes.
- l'élément de conduit est fixé solidairement au reste du couvercle, par exemple encliqueté ou emboité.
- l'extrémité libre de l'élément de conduit est munie d'une bouche de sortie dirigée vers le fond de la cuve, c'est-à-dire que l'extrémité libre de l'élément de conduit est recourbée vers le fond de la cuve, donc dirigée vers la surface de l'eau contenue dans la cuve.
- l'extrémité libre est munie d'une bouche de sortie débouchant dans le volume interne de la cuve, en particulier dans le ciel gazeux surmontant l'eau contenue dans la cuve.
- l'extrémité libre de l'élément de conduit est munie d'une bouche de sortie comprenant des échancrures permettant de diffuser largement le flux de gaz entrant vers la surface de l'eau contenue dans la cuve.
- l'élément de conduit est tubulaire.
- l'élément de conduit comprend un passage interne de gaz s'étendant entre l'orifice d'entrée de gaz et la bouche de sortie.
- l'élément de conduit comprend un premier tronçon linéaire, un premier tronçon coudé, un second tronçon linéaire et un tronçon coudé successifs.
- le couvercle comprend en outre une chambre de sortie de gaz délimitée, en haut, par le toit, c'est-à-dire la paroi externe, du couvercle et, en bas, une paroi-déflecteur interne, ladite chambre de sortie étant en communication fluidique avec le volume interne de la cuve et comprenant en outre l'orifice de sortie de gaz, ladite paroi-déflecteur interne étant située entre l'élément de conduit et le toit du couvercle. En d'autres termes, le toit du couvercle comprenant les portions de toit inclinées et plane forme le plafond de la chambre de sortie de gaz, alors que la paroi-déflecteur forme le sol de la chambre de sortie de gaz.
- la paroi-déflecteur est agencée face à la plaque métallique située dans le fond de la cuve, c'est-à-dire qu'elles peuvent être parallèles ou inclinées l'une par rapport à l'autre.
- la chambre de sortie de gaz comprend une ou plusieurs fenêtres d'entrée de gaz, encore appelées « ouïes », mettant en communication fluidique la chambre de sortie de gaz avec le volume interne de la cuve, la ou lesdites fenêtres d'entrée de gaz étant situées entre la paroi-déflecteur et la paroi externe du couvercle, c'est-à-dire de préférence agencée(s) latéralement dans la chambre de sortie de gaz.
- la chambre de sortie de gaz comprend deux fenêtres d'entrée de gaz, de préférence, les deux fenêtres d'entrée de gaz sont séparées par l'élément de conduit.
- l'humidificateur de gaz est chauffant et permet d'humidifier un gaz.
- l'humidificateur comprend un élément chauffant et une première plaque métallique, ledit élément chauffant étant en contact avec la première plaque métallique qui est elle-même en contact avec une seconde plaque métallique située dans le fond de la cuve, lorsque la cuve est agencée dans l'humidificateur.
- un élément chauffant et une autre plaque métallique (appelée « première plaque métallique ») sont agencés dans le fond du boitier.
- un capot comprenant un conduit d'évacuation de gaz humidifié en communication fluidique avec l'orifice de sortie de gaz du réservoir de l'humidificateur.
- le capot est solidaire du boitier de l'humidificateur.
- le capot comprenant un conduit d'évacuation de gaz humidifié comprenant au moins un joint d'étanchéité, de préférence un joint torique.
- le capot coopère avec le couvercle du réservoir de l'humidificateur.
- l'humidificateur de gaz sert à humidifier le gaz, typiquement de l'air, fourni par la micro-soufflante électrique grâce à de la vapeur d'eau générée dans humidificateur par chauffage au moyen de l'élément chauffant et des deux plaques métalliques.
- l'élément chauffant de l'humidificateur de gaz est commandé par des moyens de commande du ventilateur médical de manière à chauffer la première plaque métallique en réponse à un signal de commande de chauffe délivré par les moyens de commande du ventilateur médical.
- les moyens de commande du ventilateur médical commande en outre la micro-soufflante électrique pour fournir du gaz à l'humidificateur (24), typiquement de l'air.

L'invention va maintenant être mieux comprise grâce à la description détaillée suivante, faite à titre illustratif mais non limitatif, en référence aux figures annexées parmi lesquelles :
- la Figure 1 schématise un ensemble ventilateur/humidificateur de gaz médical,
- la Figure 2 est une vue en coupe longitudinale d'un mode de réalisation d'un réservoir d'eau pour un ensemble ventilateur/humidificateur de gaz médical selon l'invention, représenté en position verticale,
- la Figure 3 montre la partie haute du réservoir de la Figure 2 équipé du couvercle de la Figure 4 et une partie de l'humidificateur dans lequel s'insère le réservoir,
- la Figure 4 schématise un mode de réalisation du couvercle à toit en « V » du réservoir des Figures 2 et 3,
- la Figure 5 est une vue en perspective du réservoir d'eau de la Figure 2 sans son couvercle, et
- la Figure 6 représente le réservoir avec son couvercle à toit en « V » inséré dans un humidificateur de gaz.

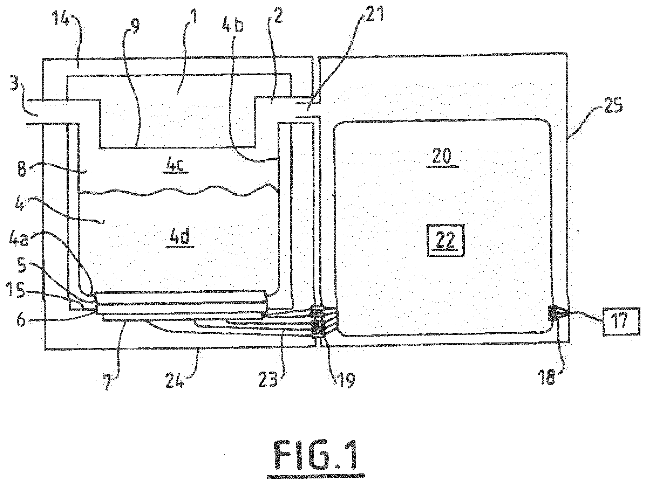

La Figure 1 schématise un mode de réalisation d'un ensemble ventilateur médical/humidificateur de gaz médical comprenant un ventilateur médical 25, c'est-à-dire un appareil de fourniture de gaz d'assistance respiratoire, et un humidificateur de gaz 24 en communication fluidique (en 21) et électrique (en 19) l'un avec l'autre.

Plus précisément, le ventilateur médical 25 comprend des moyens de fourniture de gaz, telle une micro-soufflante électrique, encore appelée « turbine » ou « compresseur », permettant de fournir un flux gazeux, par exemple de l'air, à l'humidificateur 24, et des moyens de commande 20, 22, comprenant une carte électronique 20 à (micro)processeur 22.

De préférence, les moyens de commande 20, 22 pilotent la micro-soufflante pour fournir le flux gazeux. Le flux de gaz délivré par les moyens de fourniture de gaz du ventilateur médical 25 est acheminé, via un circuit de gaz interne du ventilateur comprenant un ou plusieurs conduits de gaz ou analogue, jusqu'à une sortie de gaz 21, tel un connecteur pneumatique, assurant une communication fluidique entre le ventilateur médical 25 et l'humidificateur 24 de gaz.

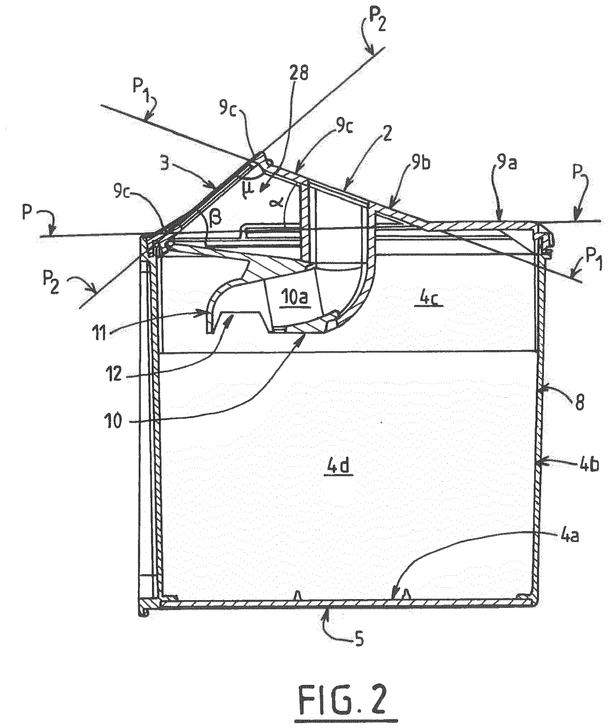

Par ailleurs, l'humidificateur de gaz 24 comprend un boitier 14 définissant un volume ou logement interne 1 destiné à recevoir un réservoir à eau 4 comprenant un bac ou cuve 8 destiné à contenir de l'eau 4d servant à humidifier le flux de gaz provenant du ventilateur 25. La cuve 8 comprend un couvercle 9, de préférence amovible, fermant le haut de la cuve 4, comme visible sur la Figure 2.

La cuve 8 du réservoir à eau 4 comprend une entrée 2 de gaz sec par laquelle le gaz à humidifier provenant du ventilateur 25 pénètre dans le réservoir à eau 4 et une sortie 3 de gaz humidifié par laquelle le gaz humidifié au sein de la cuve 8 ressort du réservoir à eau 4 et est ensuite envoyé vers le patient, via une conduite de gaz flexible par exemple reliée à une interface patient de distribution de gaz, tel un masque respiratoire ou analogue.

L'humidificateur de gaz 24 comprend en outre un élément chauffant 7 et une première plaque métallique 6 qui sont agencés dans le fond du boitier 14 de l'humidificateur 24. L'élément chauffant 7 de l'humidificateur de gaz 24 est commandé par les moyens de commande 20, 22 du ventilateur médical 25 et est par ailleurs en contact avec la première plaque métallique 6 de manière à chauffer la première plaque métallique 6 en réponse à un signal de commande de chauffe délivré par les moyens de commande 20, 22 du ventilateur médical 25.

Comme visible sur la Figure 1, le réservoir à eau 4 est agencé dans le boitier 14 de l'humidificateur de gaz 24. La cuve du réservoir 4 comprend une seconde plaque métallique 5 qui est elle-même en contact avec la première plaque métallique 6 du boitier 14 de manière à ce que les calories générées par l'élément chauffant 7 et transmises à la première plaque métallique 6 puissent passer ensuite à la seconde plaque métallique 5, puis à l'eau 4d de la cuve 8 de sorte de chauffer cette eau.

Plus précisément, la seconde plaque métallique 5 est agencée dans le fond 4a de la cuve 8 du réservoir 4, lequel fond 4a définit avec la paroi périphérique 4b de la cuve 8 du réservoir 4, un volume interne 4c contenant l'eau 4d à chauffer qui sert à humidifier le gaz fourni par le ventilateur médical 25. L'eau 4d contenue dans le réservoir 4 est donc vaporisée par chauffage au contact de la seconde plaque métallique 5.

Les plaques métalliques 5, 6 sont par exemple en aluminium, en alliage d'aluminium ou en acier inoxydable.

Le ventilateur médical 25 est alimenté en courant électrique par une alimentation 17 en courant électrique, par exemple le secteur délivrant un courant ayant une tension entre 110 et 230 V. Le courant fourni par la source de courant électrique 17 est acheminé par un câble électrique venant se raccorder électriquement au ventilateur 25 au niveau d'un connecteur électrique amont 18. Le ventilateur médical 25 est quant à lui raccordé électriquement à l'humidificateur de gaz 24 par un connecteur électrique aval 19 ou analogue. Le courant électrique est ensuite acheminé depuis le connecteur électrique aval 19 jusqu'à l'élément chauffant 7 par un ou plusieurs câbles électriques 23.

Le couvercle 9 formant un toit fermant l'ouverture de la cuve 8 est rendu solidaire de la paroi périphérique 4b de la cuve 8, grâce à un système de fixation adapté, par exemple un système par emboitement ou analogue porté par la cuve 4, le couvercle 9 ou les deux. Le système de fixation assure une étanchéité fluidique entre le rebord périphérique inférieur 16 du couvercle 9 et le rebord périphérique supérieur 17 de la cuve 8. A cette fin, le rebord périphérique inférieur 16 du couvercle 9 et le rebord périphérique supérieur 17 de la cuve 8 viennent par exemple s'emboiter l'un dans l'autre. Ceci permet de rendre le couvercle 9 amovible, c'est-à-dire détachable ou démontable, ce qui permet de remplir la cuve 4 du réservoir 8 avec de l'eau, lorsqu'elle est vide.

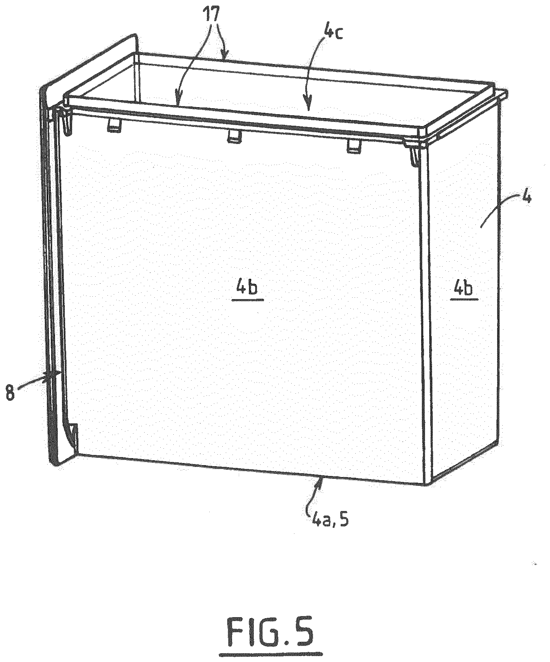

Dans le mode de réalisation des Figures 2, 3, 5 et 6, la cuve 8 à eau est parallélépipédique rectangle, c'est-à-dire ici de section rectangulaire. Elle comprend donc 4 parois se faisant face 2 à 2, i.e. parallèles 2 à 2 et perpendiculaires 2 à 2, incluant deux petites parois et deux grandes parois, les petites parois ayant une largeur inférieure à celle des grandes parois, la hauteur des petites et des grandes parois étant égale.

Selon la présente invention, afin de s'assurer que, lorsque le patient insère le réservoir 4 dans le boitier 14 de l'humidificateur 24, l'étanchéité fluidique soit garantie, notamment au niveau des orifices d'entrée 2 et de sortie 3 de gaz du réservoir 4, le couvercle 9 fermant le haut de la cuve 8 a été configuré de façon particulière, comme expliqué ci-après.

En effet, pour réduire ou éliminer les défauts d'étanchéité fluidique susceptibles de se produire au niveau des orifices d'entrée 2 et de sortie 3 de gaz qui sont situés sur le couvercle 9, il a été prévu schématiquement de configurer une partie du couvercle 9 qui forme un « toit » recouvrant et fermant la cuve 4, en forme de « V » inversé

En d'autres termes, comme détaillé en Figure 2, le couvercle 9 a été conçu de manière à présenter trois portions de toit 9a, 9b, 9c, de préférence successives, à savoir:
- une portion de toit 9a plane contenue dans un plan P qui est parallèle au fond 4a de la cuve 4, c'est-à-dire à la plaque métallique 5,
- une première portion de toit 9b plane inclinée portant l'orifice d'entrée de gaz 2 et formant un premier angle α avec le plan P, où le premier angle α est compris entre 10° et 40 °, par exemple de l'ordre de 20°, et
- une seconde portion de toit 9c plane inclinée portant l'orifice de sortie de gaz 3 et formant un second angle β avec le plan P, où le second angle β est compris entre 20 ° et 60 °, par exemple de l'ordre de 40°.

La première portion de toit 9b inclinée forme donc un premier plan P1 incliné selon le premier angle α avec le plan P, alors que la seconde portion de toit inclinée forme un second plan P2 incliné selon le second angle β avec le plan P. La première portion de toit 9b inclinée forme donc avec la seconde portion de toit inclinée, un troisième angle (µ) compris entre 90 ° et 150°, par exemple de l'ordre de 120°, comme visible sur la Figure 2.

Ces portions de toit 9b, 9c constituent donc un couvercle 9 dont une partie du toit est en « V » inversé, c'est-à-dire formé de deux portions 9a, 9b en « pente ».

Préférentiellement, les portions de toit 9a, 9b, 9c sont formées d'une seule pièce avec le reste du couvercle 9, par exemple par moulage.

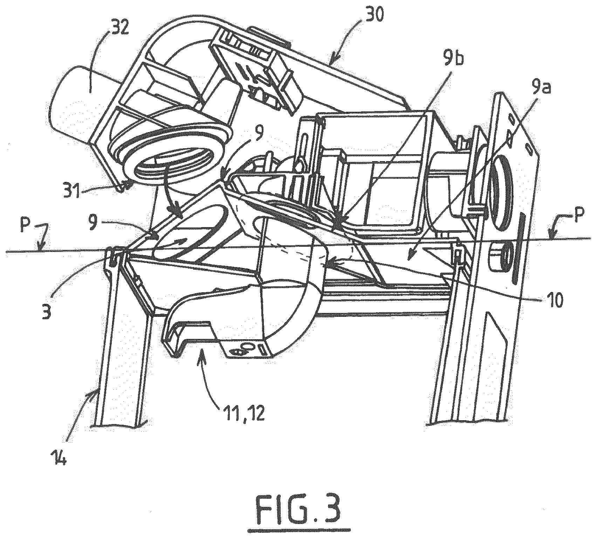

Grâce aux plans inclinés P1, P2 formant entre eux ledit toit en « V » inversé, lorsque l'utilisateur referme le capot 30 qui constitue la partie haute du boitier 14, sur le réservoir 8, ledit capot 30 exerce sur le réservoir 8 une pression à la fois verticale, pour plaquer le fond 4a à plaque métallique 6 de la cuve 4 du réservoir 8 contre l'autre plaque métallique 6 située dans le fond du boitier, elle-même en contact avec un élément chauffant 7, telle une résistance ou analogue, ce qui permet d'optimiser la transmission de chaleur (calories) vers l'eau, et horizontale, pour contraindre le ou les joints 31 portés par le capot 30 du boitier 14 et qui viennent prendre appui autour des orifices d'entrée 2 et de sortie 3 d'air du couvercle 9 en « V », comme visible en Figure 3.

La fonction d'étanchéité du chemin d'air en entrée 2 et en sortie 3 du réservoir 8, est donc renforcée par la présence des plans inclinés P1, P2 formant les première et seconde portions de toit inclinées 9b, 9c, sur lesquels viennent s'appuyer des joints d'étanchéité 31, tels des joints toriques, portés par le capot 30 formant la partie haute du boitier 14 de l'humidificateur 24.

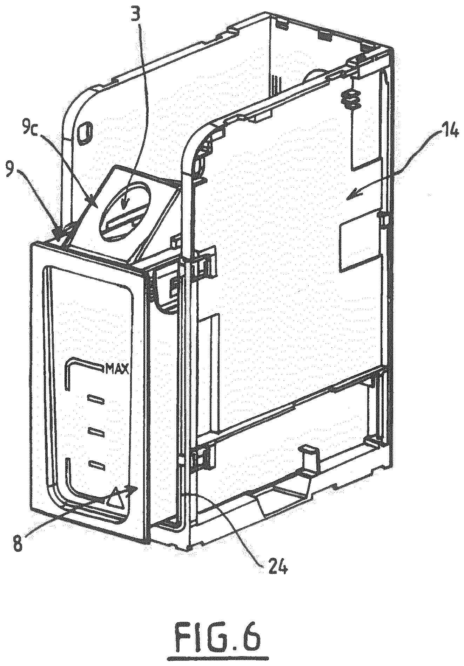

La Figure 6 illustre le réservoir 4 muni de son couvercle 9 inséré dans le boitier 14 de l'humidificateur 24 avant mise en place du capot 30 formant la partie haute du boitier 14 de l'humidificateur 24, alors que la Figure 3 montre cette mise en place du capot 30 et sa coopération avec les première et seconde portions de toit inclinées 9b, 9c, sur lesquels viennent s'appuyer le ou les joints d'étanchéité 31 portés par le capot 30.

Comme on le voit sur la Figure 3, le capot 30 peut être aussi amovible et emboitable dans le boitier 14 de sorte de former une partie de la carcasse externe de l'humidificateur 24 de gaz. Le capot 30 comprend un conduit d'évacuation de gaz humidifié 32 venant se raccorder fluidiquement à l'orifice de sortie de gaz 3, lorsque le capot 30 est fixé au boitier 14. Ce conduit d'évacuation de gaz humidifié 32 débouche dans l'orifice de sortie 3 et porte un (ou des) joint torique 31 assurant l'étanchéité au niveau de la sortie de gaz 3 portée par la deuxième portion inclinée 9c. Le conduit d'évacuation de gaz humidifié 32 permet d'évacuer le gaz humidifié provenant du réservoir 8, tel de l'air humide, vers le patient.

Par ailleurs, afin d'éviter ou de limiter les sorties intempestives d'eau du réservoir 4 de l'humidificateur 24, en particulier les retours d'eau par l'entrée de gaz 2, en direction de la micro-soufflante électrique du ventilateur médical 25, un élément de conduit creux 10, c'est-à-dire tubulaire, est agencé du côté interne du couvercle 9 de manière à être en communication fluidique avec l'orifice d'entrée 2 de gaz traversant le couvercle 9 au niveau de la première portion de toit 9b inclinée.

L'élément de conduit 10 se projette dans le volume interne 4c de la cuve 8 en direction de la paroi périphérique 4b de la cuve 8 à eau, et comprend par ailleurs une extrémité libre munie d'une bouche de sortie 11, de préférence dirigée vers le fond 4a de la cuve 8. L'élément de conduit 10 est traversé par un passage interne de gaz 10a s'étendant entre l'orifice d'entrée de gaz 2 et la bouche de sortie 11, comme illustré en Figure 2.

L'élément de conduit 10 peut être formé d'une seule pièce, par exemple par moulage, avec le reste du couvercle 9, notamment en matériau polymère, ou alors il peut être fixé solidairement au reste du couvercle 9 par exemple par emboitement ou encliquetage. L'élément de conduit 10 est coudé, c'est-à-dire que l'élément de conduit 10 comprend un premier tronçon linéaire avec l'orifice d'entrée de gaz 2, un premier tronçon coudé, un second tronçon linéaire et un tronçon coudé situé à l'extrémité libre portant la bouche de sortie 11, qui sont successifs et traversés par le passage interne 10a (cf. Fig. 2)

L'extrémité libre de l'élément de conduit 10 est ainsi munie d'une bouche de sortie 11, par laquelle arrive le gaz, tel de l'air, provenant de la micro-soufflante, la bouche de sortie 11 débouchant dans le ciel gazeux surmontant l'eau 4d contenue dans le volume interne 4c de la cuve 8, de préférence la bouche de sortie 11 est dirigée vers le fond 4a de la cuve 8 et comprend en outre des échancrures 12, ici deux échancrures, et forme dès lors une sorte de « bec », comme illustré en Figure 2.

Les échancrures 12 de la bouche de sortie 11 de l'élément de conduit 10, en élargissant le cône du flux d'air sortant par ladite bouche de sortie 11 et frappant la surface de l'eau, permettent de limiter l'amplitude des vagues se formant à la surface de l'eau, en particulier lorsque l'eau est au « niveau haut » ou proche de ce niveau, et donc de limiter la quantité d'eau rejetée vers le circuit patient, en fonctionnement normal de l'humidificateur 24.

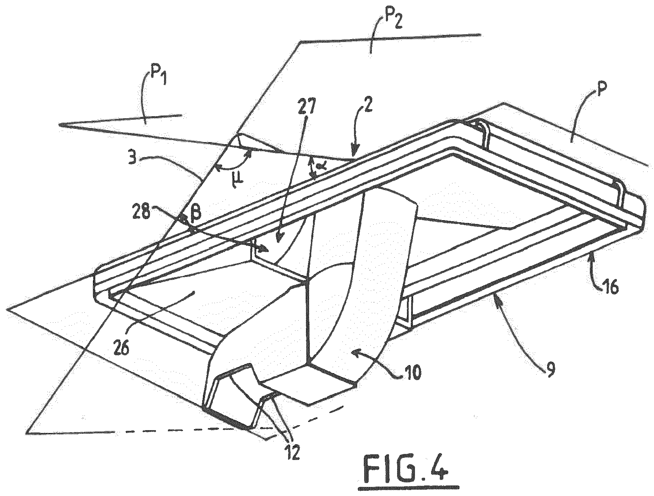

Par ailleurs, le couvercle 9 comprend aussi une chambre de sortie de gaz 28 délimitée, en haut, par la paroi externe du couvercle 9, c'est-à-dire son toit formé des portions de toit 9a, 9b, 9c, qui forme alors au moins une partie du plafond de ladite chambre de sortie de gaz 28, et une paroi-déflecteur 26 interne, en bas, qui forme alors au moins une partie du sol de ladite chambre de sortie de gaz 28. La chambre de sortie 28 est en communication fluidique, via une ou plusieurs ouvertures 27, appelées « ouïes », avec le volume interne 4c de la cuve 8 du réservoir 4 (cf. Fig. 4) et communique fluidiquement en outre avec l'orifice de sortie de gaz 3 par lequel le gaz humidifié peut être évacué vers le patient. La paroi-déflecteur 26 interne est située entre l'élément de conduit 10 et le toit 9a, 9b, 9c du couvercle 9, la paroi-déflecteur 26 étant positionnée face à la plaque métallique 5 située dans le fond 4a de la cuve 9 du réservoir 4. La paroi-déflecteur 26 interne du couvercle 9 constitue donc une sorte de plateforme située sous la sortie de gaz 3, qui permet d'éviter que de l'eau ne s'échappe directement vers la sortie de gaz 3, notamment en cas de débit d'air important provoquant des vagues à la surface de l'eau 4d.

Comme déjà mentionné, la chambre de sortie de gaz 28 comprend une ou plusieurs ouvertures ou fenêtres d'entrée de gaz 27, appelées « ouïes », mettant en communication fluidique la chambre de sortie de gaz 28 avec le volume interne 4c de la cuve 8, c'est-à-dire son ciel gazeux, même lorsque la cuve 8 est en positionnée « inclinée » ou « renversée » (Fig. 2), la ou lesdites ouïes 27 étant situées entre la paroi-déflecteur 26 et le toit du couvercle 9.

Le réservoir 4 d'eau amovible est destiné à équiper un humidificateur de gaz 24 d'un ensemble humidificateur 24/ventilateur médical 25 selon l'invention dont le ventilateur 25 est équipé d'une micro-soufflante électrique alimentant l'humidificateur 24 en gaz à humidifier, en particulier d'air.

## Revendications

1. Ensemble humidificateur de gaz/ventilateur médical (24, 25) comprenant un ventilateur (25) équipé d'une micro-soufflante électrique et un humidificateur (24) de gaz comprenant un réservoir (4) amovible et un capot (30), le réservoir (4) comprenant une cuve (8) à eau et un couvercle (9) agencé sur la cuve (8) de manière à former un toit couvrant ladite cuve (8);
- le couvercle (9) du réservoir (4) comprenant un orifice d'entrée de gaz (2), un orifice de sortie de gaz (3), une portion de toit (9a) plane contenue dans un plan (P), une première portion de toit (9b) inclinée portant l'orifice d'entrée de gaz (2) et formant un premier angle (α) avec le plan (P), où le premier angle (α) est compris entre 15° et 30°, et une seconde portion de toit (9c) inclinée portant l'orifice de sortie de gaz (3) et formant un second angle (β) avec le plan (P), où le second angle (β) est compris entre 30° et 50°,
- la cuve (8) à eau comprenant un fond (4a) muni d'une plaque métallique (5) et une paroi périphérique (4b) se projetant vers le haut à partir dudit fond (4a), ledit couvercle (9) étant solidaire de la paroi périphérique (4b) de ladite cuve (8),
- et le capot (30) comprenant un conduit d'évacuation de gaz humidifié (32) en communication fluidique avec l'orifice de sortie de gaz (3) du couvercle (9) du réservoir (8).

2. Ensemble selon la revendication 1, **caractérisé en ce que** la première portion de toit (9b) inclinée du réservoir (4) forme, avec le plan (P), un premier angle (α) compris entre 15° et 25°.

3. Ensemble selon la revendication 1, **caractérisé en ce que** la seconde portion de toit (9c) inclinée du réservoir (4) forme, avec le plan (P), un second angle (β) compris entre 35° et 45°.

4. Ensemble selon l'une des revendications précédentes, **caractérisé en ce que** le couvercle (9) du réservoir (4) formant un toit fermant la cuve (8) comprend une première portion de toit (9b) inclinée et une seconde portion de toit (9c) inclinée, lesdites première et seconde portions de toit (9b, 9c) inclinées formant entre elles un troisième angle (µ) compris entre 90 ° et 150°, de préférence entre 100° et 140°.

5. Ensemble selon l'une des revendications précédentes, **caractérisé en ce que** l'orifice d'entrée (2) de gaz du réservoir (4) est en communication fluidique avec un élément de conduit (10) se projetant dans le volume interne (4c) de la cuve (8) et comprenant un passage interne de gaz (10a) s'étendant entre l'orifice d'entrée de gaz (2) et une bouche de sortie (11) débouchant dans le volume interne (4c) de la cuve (8).

6. Ensemble selon l'une des revendications précédentes, **caractérisé en ce que** la cuve (8) à eau est un parallélépipède rectangle.

7. Ensemble selon l'une des revendications précédentes, **caractérisé en ce que** la portion de toit (9a) plane du réservoir (4) est contenue dans un plan (P) parallèle à fond de cuve (4).

8. Ensemble selon la revendication 1, **caractérisé en ce que** le conduit d'évacuation de gaz humidifié (32) comprend au moins un joint d'étanchéité (31).

9. Ensemble selon la revendication 1, **caractérisé en ce que** l'humidificateur (24) comprend un élément chauffant (7) et une première plaque métallique (6), ledit élément chauffant (7) étant en contact avec la première plaque métallique (6) qui est elle-même en contact avec une seconde plaque métallique (5) située dans le fond de la cuve (8), lorsque la cuve (8) est agencée dans l'humidificateur (24).

10. Ensemble selon la revendication 9, **caractérisé en ce que** l'élément chauffant (7) et la première plaque métallique (6) sont agencés dans le fond du boitier (14) de l'humidificateur (24).

11. Ensemble selon l'une des revendications 9 ou 10, **caractérisé en ce que** l'élément chauffant (7) de l'humidificateur de gaz (24) est commandé par des moyens de commande (20, 22) du ventilateur médical (25) de manière à chauffer la première plaque métallique (6) en réponse à un signal de commande de chauffe délivré par les moyens de commande (20, 22) du ventilateur médical (25).

12. Ensemble selon la revendication 9, **caractérisé en ce que** les moyens de commande (20, 22) du ventilateur médical (25) commande en outre la micro-soufflante électrique pour fournir du gaz à l'humidificateur (24).

## Patentansprüche

1. Einheit aus Gasbefeuchter/medizinischem Beatmungsgerät (24, 25), umfassend ein Beatmungsgerät (25), das mit einem elektrischen Mikrogebläse ausgestattet ist, und einen Gasbefeuchter (24), der einen abnehmbaren Behälter (4) und eine Haube (30) umfasst, wobei der Behälter (4) einen Wassertank (8) und eine Abdeckung (9) umfasst, die so auf dem Tank (8) angeordnet ist, dass sie ein Dach bildet, das den Tank (8) bedeckt;
- wobei die Abdeckung (9) des Behälters (4) eine Gaseingangssöffnung (2), eine Gasauslassöffnung (3), einen ebenen Dachabschnitt (9a), der in einer Ebene (P) enthalten ist, einen ersten geneigten Dachabschnitt (9b), der die Gaseingangssöffnung (2) trägt und einen ersten Winkel (α) mit der Ebene (P) bildet, wobei der erste Winkel (α) zwischen 15° und 30° enthalten ist, und einen zweiten geneigten Dachabschnitt (9c) umfasst, der die Gasauslassöffnung (3) trägt und mit der Ebene (P) einen zweiten Winkel (β) bildet, wobei der zweite Winkel (β) zwischen 30° und 50° enthalten ist,
- wobei der Wassertank (8) einen mit einer Metallplatte (5) versehenen Boden (4a) und eine von dem Boden (4a) nach oben ragende Umfangswand (4b) umfasst, wobei die Abdeckung (9) mit der Umfangswand (4b) des Tanks (8) fest verbunden ist,
- und wobei die Haube (30) eine Auslassleitung des befeuchteten Gases (32) in fluidischer Verbindung mit der Gasauslassöffnung (3) der Abdeckung (9) des Behälters (8) umfasst.

2. Einheit nach Anspruch 1, **dadurch gekennzeichnet, dass** der erste geneigte Dachabschnitt (9b) des Behälters (4) mit der Ebene (P) einen ersten Winkel (α) bildet, der zwischen 15° und 25° enthalten ist.

3. Einheit nach Anspruch 1, **dadurch gekennzeichnet, dass** der zweite geneigte Dachabschnitt (9c) des Behälters (4) mit der Ebene (P) einen zweiten Winkel (β) bildet, der zwischen 35° und 45° enthalten ist.

4. Einheit nach einem der vorstehenden Ansprüche, **dadurch gekennzeichnet, dass** die Abdeckung (9) des Behälters (4), die ein den Tank (8) schließendes Dach bildet, einen ersten geneigten Dachabschnitt (9b) und einen zweiten geneigten Dachabschnitt (9c) umfasst, wobei der erste und zweite geneigte Dachabschnitt (9b, 9c) zwischen sich einen dritten Winkel (µ) bilden, der zwischen 90° und 150°, bevorzugt zwischen 100° und 140° enthalten ist.

5. Einheit nach einem der vorstehenden Ansprüche, **dadurch gekennzeichnet, dass** die Gaseingangssöffnung (2) des Behälters (4) in fluidischer Verbindung mit einem Leitungselement (10) steht, das in das innere Volumen (4c) des Tanks (8) ragt und einen internen Gasdurchlass (10a) umfasst, der sich zwischen der Gaseingangssöffnung (2) und einer Auslassmündung (11) erstreckt, die in das innere Volumen (4c) des Tanks (8) mündet.

6. Einheit nach einem der vorstehenden Ansprüche, **dadurch gekennzeichnet, dass** der Wassertank (8) ein rechteckiger Quader ist.

7. Einheit nach einem der vorstehenden Ansprüche, **dadurch gekennzeichnet, dass** der ebene Dachabschnitt (9a) des Behälters (4) in einer Ebene (P) parallel zu dem Tankboden (4) enthalten ist.

8. Einheit nach Anspruch 1, **dadurch gekennzeichnet, dass** die Auslassleitung des befeuchteten Gases (32) mindestens einen Abdichtring (31) umfasst.

9. Einheit nach Anspruch 1, **dadurch gekennzeichnet, dass** der Befeuchter (24) ein Aufheizelement (7) und eine erste Metallplatte (6) umfasst, wobei das Aufheizelement (7) mit der ersten Metallplatte (6) in Kontakt steht, die ihrerseits mit einer zweiten Metallplatte (5) in Kontakt steht, die sich am Boden des Tanks (8) befindet, wenn der Tank (8) im Befeuchter (24) angeordnet ist.

10. Einheit nach Anspruch 9, **dadurch gekennzeichnet, dass** das Aufheizelement (7) und die erste Metallplatte (6) im Boden des Gehäuses (14) des Befeuchters (24) angeordnet sind.

11. Einheit nach einem der Ansprüche 9 oder 10, **dadurch gekennzeichnet, dass** das Aufheizelement (7) des Gasbefeuchters (24) so durch Steuermittel (20, 22) des medizinischen Beatmungsgeräts (25) gesteuert wird, dass die erste Metallplatte (6) als Reaktion auf ein von den Steuermitteln (20, 22) des medizinischen Beatmungsgeräts (25) abgegebenes Aufheizssteuersignal aufgeheizt wird.

12. Einheit nach Anspruch 9, **dadurch gekennzeichnet, dass** die Steuermittel (20, 22) des medizinischen Beatmungsgeräts (25) weiter das elektrische Mikrogebläse steuern, um dem Befeuchter (24) Gas zuzuführen.

## Claims

1. Gas humidifier/medical ventilator assembly (24, 25) comprising a ventilator (25) equipped with an electric micro-fan and a gas humidifier (24) comprising a removable tank (4) and a cap (30), the tank (4) comprising a water container (8) and a cover (9) arranged on the container (8) so as to form a roof covering said container (8);
- the cover (9) of the tank (4) comprising a gas inlet orifice (2), a gas outlet orifice (3), a flat roof portion (9a) contained in a plane (P), a first inclined roof portion (9b) carrying the gas inlet orifice (2) and forming a first angle (α) with the plane (P), where the first angle (α) is of between 15° and 30°, and a second inclined roof portion (9c) carrying the gas outlet orifice (3) and forming a second angle (P) with the plane (P), where the second angle (β) is of between 30° and 50°,
- the water container (8) comprising a bottom (4a) equipped with a metal plate (5) and a peripheral wall (4b) being projected towards the top from said bottom (4a), said cover (9) being integral with the peripheral wall (4b) of said container (8),
- and the cap (30) comprising a humidified gas evacuation conduit (32) in fluid communication with the gas outlet orifice (3) of the cover (9) of the tank (8).

2. Assembly according to claim 1, **characterised in that** the first inclined roof portion (9b) of the tank (4) forms, with the plane (P), a first angle (α) of between 15° and 25°.

3. Assembly according to claim 1, **characterised in that** the second inclined roof portion (9c) of the tank (4) forms, with the plane (P), a second angle (β) of between 35° and 45°.

4. Assembly according to one of the preceding claims, **characterised in that** the cover (9) of the tank (4) forming a roof closing the container (8) comprises a first inclined roof portion (9b) and a second inclined roof portion (9c), said first and second inclined roof portions (9b, 9c) together form a third angle (µ) of between 90° and 150°, preferably between 100° and 140°.

5. Assembly according to one of the preceding claims, **characterised in that** the gas inlet orifice (2) of the tank (4) is in fluid communication with a conduit element (10) being projected into the inner volume (4c) of the container (8) and comprising an inner gas passage (10a) extending between the gas inlet orifice (2) and an outlet mouth (11) opening into the inner volume (4c) of the container (8).

6. Assembly according to one of the preceding claims, **characterised in that** the water container (8) is a rectangular parallelepiped.

7. Assembly according to one of the preceding claims, **characterised in that** the flat roof portion (9a) of the tank (4) is contained in a plane (P) parallel to the container bottom (4).

8. Assembly according to claim 1, **characterised in that** the humidified gas evacuation conduit (32) comprises at least one seal (31).

9. Assembly according to claim 1, **characterised in that** the humidifier (24) comprises a heating element (7) and a first metal plate (6), said heating element (7) being in contact with the first metal plate (6) which is itself in contact with a second metal plate (5) situated in the bottom of the container (8), when the container (8) is arranged in the humidifier (24).

10. Assembly according to claim 9, **characterised in that** the heating element (7) and the first metal plate (6) are arranged in the bottom of the casing (14) of the humidifier (24).

11. Assembly according to one of claims 9 or 10, **characterised in that** the heating element (7) of the gas humidifier (24) is controlled by means for controlling (20, 22) the medial ventilator (25) so as to heat the first metal plate (6) in response to a heating control signal delivered by the means for controlling (20, 22) the medical ventilator (25).

12. Assembly according to claim 9, **characterised in that** the control means (20, 22) of the medical ventilator (25) furthermore controls the electric micro-fan to supply gas to the humidifier (24).
